(19) Europäisches Patentamt European Patent Office Office européen des brevets

(11) **EP 2 413 991 B1**

(12) **EUROPÄISCHE PATENTSCHRIFT**

(45) Veröffentlichungstag und Bekanntmachung des Hinweises auf die Patenterteilung:
**02.05.2018 Patentblatt 2018/18**

(21) Anmeldenummer: **10712361.4**

(22) Anmeldetag: **31.03.2010**

(51) Int Cl.:
**A61M 1/36** (2006.01)     **A61M 1/16** (2006.01)

(86) Internationale Anmeldenummer:
**PCT/EP2010/002086**

(87) Internationale Veröffentlichungsnummer:
**WO 2010/112223 (07.10.2010 Gazette 2010/40)**

(54) **VORRICHTUNG ZUR ERMITTLUNG UND REGELUNG DER KONZENTRATION MINDESTENS EINES GELÖSTEN STOFFES IN EINEM FLUIDKREISLAUF**

DEVICE FOR DETERMINING AND CONTROLLING THE CONCENTRATION OF AT LEAST ONE SOLUTE IN A FLUID CIRCUIT

DISPOSITIF POUR DÉTERMINER ET RÉGULER LA CONCENTRATION D'AU MOINS UNE SUBSTANCE DISSOUTE DANS UN CIRCUIT DE FLUIDE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **31.03.2009 DE 102009015649**

(43) Veröffentlichungstag der Anmeldung:
**08.02.2012 Patentblatt 2012/06**

(73) Patentinhaber: **Fresenius Medical Care Deutschland GmbH**
**61352 Bad Homburg (DE)**

(72) Erfinder:
• **GROSS, Malte**
**89081 Ulm (DE)**

• **MAIERHOFER, Andreas**
**97422 Schweinfurt (DE)**

(74) Vertreter: **Herrmann, Uwe et al**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
**EP-A2- 0 272 414     EP-A2- 0 330 892**
**US-A- 4 508 622     US-A1- 2001 004 523**

**Beschreibung**

Die vorliegende Erfindung betrifft eine Vorrichtung zur Ermittlung und Regelung der Konzentration wenigstens eines gelösten Stoffes in einem Fluidkreislauf, wobei der Fluidkreislauf aus wenigstens zwei Teilkreisläufen besteht und wobei die Teilkreisläufe durch einen Filter semipermeabel getrennt sind. Fluidkreislaufsysteme, die aus zwei Teilkreisläufen bestehen, wobei ein Teilkreislauf z. B. schwer zugänglich, eine Zugänglichkeit des Teilkreislaufs unerwünscht oder der Teilkreislauf sogar unzugänglich ist und/oder Sensoren und Aktoren zur Bestimmung von gelösten Stoffen und zur Beeinflussung derselben nur am zugänglichen Teilkreislauf angebracht werden können, stellen den Anwender zumeist dann vor ein Problem, wenn die Konzentration eines gelösten Stoffes im zugänglichen Teilkreislauf auf einen Sollwert in Abhängigkeit vom Ist- oder Sollwert des gelösten Stoffes im unzugänglichen Teilkreislauf eingeregelt und/oder konstant gehalten werden soll, jedoch der Istwert des gelösten Stoffes im unzugänglichen Teilkreislauf beispielsweise nicht oder nur mit unvertretbarem Aufwand ermittelbar und/oder unbekannt ist.

Dieses Problem tritt in vielen technischen Bereichen auf, insbesondere in der Filtrierungstechnik, wobei von einem Teilkreislauf zu einem anderen Teilkreislauf ein Austausch gelöster Stoffe über eine semipermeable Membran erfolgen soll.

Insbesondere stellt sich dieses Problem in der Dialysetechnik, denn dort kann zwar im Dialysekreislauf eine Überwachung und Beeinflussung der Konzentration von gelösten Stoffen wie Calcium-Ionen, Natrium-Ionen, Chlorid-Ionen usw. durchgeführt werden. Dies ist jedoch im Blutkreislauf wenn überhaupt nur mit großem Aufwand möglich und größtenteils sogar aus hygienischen Gründen unerwünscht.

Im Falle der isonaträmischen Dialyse lässt sich das vorstehend umrissene Problem gut illustrieren:
Im gesunden Menschen wird die Natriumkonzentration im Körper im Wesentlichen durch die Niere durch die Regulation der Elektrolyt- und Wasserausscheidungen in engen Grenzen auf einen individuenspezifischen Wert konstant gehalten. Nach der Setpoint-Hypothese halten auch Dialysepatienten mit terminalem Nierenversagen ihre Natriumkonzentration weitgehend konstant. Da aber bei ihnen eine Wasser- und Natrium-Ausscheidung zwischen den Dialysebehandlungen nicht möglich ist, bedeutet dies, dass jede aufgenommene Salzmenge wegen des einsetzenden Durstgefühls eine Aufnahme von Wasser nach sich zieht.

Bei einer typischen Hämodialysebehandlung erfolgt der Großteil des Salztransfers konvektiv durch die Ultrafiltration, da hierdurch dem Patienten Flüssigkeit entzogen wird, deren Elektrolytgehalt dem des Plasmawassers des Patienten entspricht. Im Idealfall entspricht diese Menge der Salzmenge, die der Patient im Zeitraum zwischen zwei Dialysebehandlungen zu sich genommen hat. Üblicherweise wird dem Patienten mittels Ultrafiltration die Menge an Flüssigkeit entzogen, die er zwischen den Dialysebehandlungen aufgenommen hat. Durch die Ultrafiltration ändert sich die Salzkonzentration im Patienten nicht. Dagegen kommt es durch Diffusion im Dialysator zu einem Elektrolyttransfer, der eine Änderung der Salzkonzentration im Patienten zur Folge haben kann.

Liegt die Salzkonzentration im Dialysat deutlich über derjenigen im Patienten, so wird dem Patienten während der Dialysebehandlung durch Diffusion Salz zugeführt. Dieses geschieht in der Praxis häufig entweder unwissentlich, da die Salzkonzentration meist nicht individuell angepasst wird, oder absichtlich, um die hämodynamische Stabilität während der Dialysebehandlung zu verbessern.

Jedoch wird der Patient nach der Dialysebehandlung die über seinem Setpoint liegende Konzentration durch Trinken wieder ausgleichen, so dass eine zu hohe Salzkonzentration während der Dialysebehandlung zu einer erhöhten interdialytischen Flüssigkeitsaufnahme und damit zu verstärkter Überwässerung und ggf. zu chronischem Bluthochdruck führt.

Um die vorstehenden Probleme zu vermeiden, ist es medizinisch sinnvoll, eine isonaträmische Dialyse durchzuführen. Eine isonaträmische Dialyse ist eine Dialyse, bei der dem Patienten kein Natrium über die Membran des Dialysators diffusiv zugeführt oder entnommen wird. Eine Methode hierzu könnte sein, das Plasma-Natrium zu Beginn der Behandlung im Kliniklabor oder mittels eines Blutgasanalysators zu bestimmen und die Natriumkonzentration des Dialysats auf einen Wert einzustellen, der die Natriumkonzentration im Blut nicht durch Diffusion über die Filtermembran verändert. Hierbei ist der Donnan-Effekt zu berücksichtigen, der dazu führt, dass die Natriumkonzentrationen im Blut und im Dialysat bei isonaträmischer Dialyse unterschiedlich sind.

Neben den Problemen einer Blutprobenentnahme wie Personalaufwand und Laborkosten treten hier weitere prinzipielle Probleme auf. Denn bedingt durch die begrenzte Messgenauigkeit der Labormethoden kann der gemessene Wert leicht um bis zu 3 mmol/l vom tatsächlichen Wert abweichen. Ferner zeigen Blutgasanalysator und Kliniklabor systematisch unterschiedliche Werte, da das eine Verfahren eine direkte und das andere Verfahren eine indirekte Potentiometrie zu Grunde liegen hat. Ferner weisen die Dialysatkonzentrate Produktionstoleranzen auf, die zu Ungenauigkeiten der Natrium-Konzentration im Dialysat führen könnten. Zusätzlich ist ein Ausgleich der Toleranzen in der Natrium-Konzentration im Dialysekonzentrat durch Messung derselben nur schwer möglich, da eine exakte Messung der Natrium-Konzentration im Dialysat mit den auf Blut kalibrierten Analysatoren praktisch nicht möglich ist.

Daher bliebe als einzige Möglichkeit zur Erreichung einer isonaträmischen Dialyse, die Plasma-Natrium-Konzentration vor und nach der Dialyse in Blutproben zu bestimmen und die Natrium-Einstellung des Dialysats in einer

EP 2 413 991 B1

Charge so lange zu variieren, bis die Isonatriämie erreicht ist. Dieses Verfahren ist jedoch für die Praxis nicht realisierbar, so dass als Konsequenz alle Patienten eines Dialysezentrums im Wesentlichen mit dem gleichen Dialysat-Natrium behandelt werden.

Auch die sog. bilanzneutralen Natrium-Profile des Dialysegeräts helfen hier nicht, da sich die Bilanzneutralität nur darauf bezieht, dass unter Zugrundelegung eines Modells der Salztransfer über den Dialysator bei Verwendung des Profils demjenigen eines bestimmten festen Dialysatnatriums entspricht.

Ferner können medizinische Erwägungen eine kontrollierte Abweichung von einer isonaträmischen Dialyse erfordern. Dieses kann z. B. dann der Fall sein, wenn eine Elektrolytentgleisung vorliegt, so dass am aktuellen Behandlungstag die Setpoint-Hypothese des Patienten nicht erfüllt ist. Ebenso ist bei der Behandlung eine Hyponatriämie eine Erhöhung der Plasma-Konzentration sinnvoll. Umgekehrt kann bei Patienten, die zu übermäßiger Flüssigkeitsaufnahme zwischen den Dialysebehandlungen neigen, ein Absenken des Plasma-Natriums unter den Setpoint sinnvoll sein, um dadurch das Durstgefühl und damit die Flüssigkeitsaufnahme des Patienten zu reduzieren.

Aus dem Stand der Technik sind bereits Lösungsansätze für die vorstehend aufgezeigten Probleme bekannt, die jedoch noch nicht zufriedenstellend sind.

So ist aus der Druckschrift Petitclerc et al., "Sodium management in dialysis by conductivity", Advances in renal replacement therapy, Vol 6, No 3 (July), 1999: pp 243-254, ein Verfahren zu Bestimmung des Natriumgehaltes im Blut auf der Grundlage einer Leitfähigkeitsmessung des Dialysats bekannt. Dabei macht man sich zu Nutze, dass es über den Elektrolytaustausch zwischen Blut und Dialysat über den Filter zu einem Elektrolytausgleich kommt, wenn ein Konzentrationsunterschied von Elektrolyten besteht. Über den Leitfähigkeitsunterschied des Dialysats stromauf und stromab des Filters kann detektiert werden, ob es zu einem Elektrolyttransfer zwischen Dialysat und Blut gekommen ist. Da es während der Dialyse nicht nur zum Natriumaustausch über die Filtermembranen kommt, sondern z. B. auch Kalium entzogen und Bicarbonat zur Korrektur der Azidose zugeführt werden kann, neben dem Austausch anderer Elektrolyte wie Calcium, Chlor etc. und Nichtelektrolyte wie Glucose und Harnstoff, lässt sich jedoch der Natriumgehalt des Blutes nicht direkt aus der Leitfähigkeit des Dialysats bestimmen.

Die US 4,923,613 beschreibt ein Verfahren und eine Maschine zur Bestimmung des Natriumgehaltes des Blutes eines Dialysepatienten durch Verändern der Dialysatzusammensetzung ab einem Zeitpunkt $t$ derart, dass sich nach einer Dialysatlaufzeit $t+t_i$ stromabwärts der Dialysatseite des Dialysefilters die gleiche Leitfähigkeit einstellt wie zu der Zeit t stromaufwärts der Dialysatseite des Dialysefilters und dass sich aus dieser Leitfähigkeit der Natriumgehalt des Blutes bestimmen lässt.

Die US 4,508,622 A offenbart ein Dialysegerät mit einer Einheit zur Herstellung des Dialysats und einem Dialysefilter. Das Gerät umfasst einen ersten Detektor stromaufwärts des Dialysefilters und einen zweiten Detektor stromabwärts des Dialysefilters auf der Dialysatseite. Die Messwerte der Detektoren werden verglichen und letztendlich dazu verwendet, die Zusammensetzung des Dialysats zu bestimmen. Das Konzept der US 4,508,622 A richtet sich insbesondere an Dialysegeräte mit Dialysefiltern, die eine hohe Austauschkapazität aufweisen, da die Dialysatzusammensetzung unmittelbar und jederzeit an die Anforderungen des Patienten angepasst werden kann.

Es ist daher die Aufgabe der vorliegenden Erfindung, eine Vorrichtung zu Regelung der Konzentration wenigstens eines gelösten Stoffes in einem Fluidkreislauf der eingangs genannten Art in vorteilhafter Weise weiterzubilden, insbesondere dahingehend, dass diese eine verbesserte und sicherere Ermittlung der Konzentrationsänderung eines gelösten Stoffes in einem schwer zugänglichen oder unzugänglichen Teilkreislauf und die Regelung eines gelösten Stoffes, vorzugsweise auf einen initial unbekannten Istwert und/oder Sollwert des gelösten Stoffes im unzugänglichen Teilkreislauf, ermöglicht. Zusätzlich erlaubt die Erfindung eine verbesserte und sicherere Ermittlung der Konzentration des gelösten Stoffes im unzugänglichen Teilkreislauf.

Diese Aufgabe wird erfindungsgemäß durch eine Vorrichtung zur Ermittlung und Regelung der Konzentration wenigstens eines gelösten Stoffes in einem Fluidkreislauf mit den Merkmalen des Anspruchs 1 gelöst. Vorteilhafte Ausführungen der Erfindung sind Gegenstand der abhängigen Ansprüche 2 bis 12.

Demnach ist eine bevorzugte Ausführungsform eine Vorrichtung, die für einen Fluidkreislauf einer Dialysemaschine vorgesehen ist. Dieser Fluidkreislauf besteht aus einem Dialysatkreislauf, im Folgenden auch zugänglicher erster Teilkreislauf genannt, und einem Blutkreislauf, im Folgenden auch unzugänglicher zweiter Teilkreislauf genannt, die semipermeabel von wenigstens einem Filter voneinander getrennt sind. Die Vorrichtung umfasst erste und zweite Mittel zur mittelbaren oder unmittelbaren Detektion der Konzentration wenigstens eines gelösten Stoffes im Dialysatkreislauf vor und nach dem Filter. Die Vorrichtung umfasst weiterhin eine Ermittlungseinheit, die unter Verwendung von zuvor eingegebenen patientenspezifischen Daten und den ermittelten oder bekannten Konzentrationen wenigstens eines gelösten Stoffes im Dialysatkreislauf vor und nach dem Filter die Konzentration eines zu regelnden gelösten Stoffes im Blutkreislauf und Dialysatkreislaufs ermittelt. Die Vorrichtung umfasst weiterhin Mittel zur Regelung der Änderung der Konzentration des zu regelnden gelösten Stoffes im Blutkreislauf auf einen zuvor eingegebenen Sollwert, oder dynamisch auf einen Verlauf von bestimmten Werten. Die Vorrichtung umfasst weiterhin Mittel zum Einlesen zuvor abgespeicherter Daten von einem Speichermedium, vorzugsweise eine patientenindividuelle Speicherkarte, und Mittel zum Speichern von Daten auf dieses Speichermedium.

Vorzugsweise handelt es sich bei dem Sollwert um den Sollwert der Konzentrationsänderung des gelösten Stoffes im Blutkreislauf, der meist schlecht zugänglich ist, oder aus Hygienegründen nicht mit Sensoren versehen werden soll.

Die Ermittlungseinheit kann vorteilhafter Weise ein Regelungsmodell aufweisen, dass das Verhalten der gelösten Stoffe im Dialysatkreislauf und im vaskulären Blutkreislauf des Patienten, das Übertrittsverhalten der gelösten Stoffe über die semipermeable Membran, die zeitliche Änderung und/oder initialen Startwerte für die iterative Sollwertermittlung unter Berücksichtigung patientenindividueller Daten, sowie der Behandlungsparameter, abbildet bzw. umfasst.

Beispielsweise kann somit der Unterschied der Konzentration des zu regelnden Stoffes stromauf- und stromabwärts des Filters über den mittelbar oder unmittelbar bestimmten Konzentrationsunterschied des gleichen und/oder eines und/oder mehrerer anderer Stoffe im Dialysatkreislauf ermittelt werden und durch Mittel zur Änderung der Konzentration eines oder mehrerer Stoffe im Dialysat stromaufwärts des Filters eingeregelt werden. Hierbei ist es ein besonders bevorzugtes Ziel der Regelung, dass sich die Konzentration des zu regelnden Stoffes im vaskulären Blutkreislauf des Patienten während der Behandlung nicht ändert. Ein weiteres bevorzugtes Ziel der Regelung ist die stetige Änderung der Konzentration des zu regelnden Stoffes im vaskulären Blutkreislauf des Patienten über die Dauer der Behandlung um einen vom Arzt eingegebenen Wert, der eine Konzentrationserhöhung oder eine Konzentrationsverminderung sein kann.

Dabei kann vorgesehen sein, dass sich die Ermittlungseinheit dynamisch anpassen kann und z. B. Veränderungen in den Randbedingungen wie Konzentrationsverschiebungen aufnehmen kann. Vorzugsweise geht die dynamische Anpassbarkeit der Ermittlungseinheit soweit, dass die Ermittlungseinheit als lernendes und/oder selbstadaptierendes System ausgebildet ist. Besonders vorteilhaft ist, dass auch ohne Eingabe eines Sollwertes für die Konzentration eines gelösten Stoffes im vaskulären Blutkreislauf eine Regelung des gelösten Stoffes im Blutkreislauf erfolgt, und zwar mit der ermittelten Konzentrationsänderung eines gelösten Stoffes im Dialysatkreislauf vor und nach dem Filter und unter Verwendung des vorgehaltenen Regelungsmodells, welches ohne bekannte absolute Konzentration des zu regelnden Stoffes im vaskulären Blutkreislauf des Patienten auf die Änderung der Konzentration des zu regelnden Stoffes im Blut einregelt, die vorzugsweise Null ist.

Das vorgehaltene Regelungsmodell ist dabei vorteilhafterweise derart ausgestaltet, dass es die Gesetzmäßigkeiten der Konzentrationsverteilung gelöster Stoffe im Blutkreislauf abbildet. Insbesondere ist es möglich, dass im Regelungsmodell zudem die Gesetzmäßigkeiten des Stoffübertritts über eine semipermeable Membran von einem zum anderen Teilkreislauf mit abgebildet sind.

Des Weiteren ist möglich, dass die Detektionseinheit keine weiteren Mittel aufweist, mit denen im zweiten Teilkreislauf die Konzentration des gelösten Stoffes bestimmbar ist.

Darüber hinaus ist denkbar, dass die ersten und zweiten Mittel Leitfähigkeitssensoren umfassen oder als solche ausgeführt sind. Leitfähigkeitssensoren weisen den Vorteil auf, dass die durch die Summe der gelösten Stoffe resultierende Leitfähigkeit sicher und zuverlässig ermittelt werden kann.

Es ist vorgesehen durch die mittels der ersten und zweiten Mittel ermittelte Leitfähigkeit mittelbar die Konzentration eines gelösten Stoffes mittels der Regelungseinheit zu berechnen. Insbesondere ist in diesem Zusammenhang daran zu denken, dass in Fällen, bei denen ein einziger gelöster Stoff in hoher Konzentration und die übrigen Stoffe in vernachlässigbarer oder konstanter Konzentration vorliegen, der interessierende Stoff ohne weiteres auch aus einem Summenwert wie z. B. der Leitfähigkeit ermittelbar ist.

Von Vorteil ist es, wenn die Einregelung nur auf eine vordefinierte Schwankungsbreite des Sollwertes im ersten oder zweiten Teilkreislauf beschränkt werden kann. Die Vorgabe einer vordefinierten Schwankungsbreite ermöglicht es, Konzentrationsentgleisungen auf zumindest einer Seite des Fluidkreislaufs während des Betriebes zu verhindern. In diesem Zusammenhang hat sich herausgestellt, dass eine Schwankungsbreite vorzugsweise innerhalb von $\pm$ 5 %, teilweise bis zu $\pm$ 10 % des Sollwertes von Vorteil ist.

Des Weiteren kann vorgesehen sein, dass eine Speichervorrichtung zur Einspeicherung eines initialen Sollwertes für den gelösten Stoff im vaskulären Blutkreislauf des Patienten und/oder im Dialysatkreislauf stromaufwärts des Filters vorgesehen ist, wobei die Speichervorrichtung vorzugsweise ein entnehmbares Speichermedium umfasst. Ein derartiges entnehmbares Speichermedium kann eine Chipkarte sein, in der beispielsweise der initiale Sollwert für z. B. Natrium im Dialysat-Teilkreislauf eingespeichert ist. Grundsätzlich ist es möglich, auch weitere Werte, wie sämtliche Konzentrationen von gelösten Stoffen sowie die daraus resultierende Leitfähigkeit einzuspeichern. Ferner können ergänzende Randbedingungen für das Regelungsmodell eingespeichert werden, um eine höhere Genauigkeit der Einregelung bzw. eine schnellere Einregelung zu ermöglichen.

Es ist denkbar, dass die Speichervorrichtung mit der Regelungseinheit in Verbindung steht.

Darüber hinaus ist es denkbar, dass es sich bei dem gelösten Stoff um Natrium-Ionen handelt. Der Fluidkreislauf besteht aus einem Blutkreislauf und einem Dialysatkreislauf einer Dialysemaschine, die semipermeabel durch wenigstens einen Filter voneinander getrennt sind. Demnach ist es vorgesehen, dass die Konzentrationsänderung eines gelösten Stoffes im vaskulären Blutkreislauf während einer Hämodialysebehandlung mittel- oder unmittelbar bestimmt wird und durch geeignete Maßnahmen eingeregelt wird. Hierzu wird der Konzentrationsunterschied des zu regelnden Stoffes im Dialysat stromauf- und stromabwärts des Filters mittel- oder unmittelbar bestimmt, wobei ein Modell zur Abbildung der Beiträge anderer als des zu regelnden Stoffes auf die mittel- oder unmittelbare Bestimmung des Konzentrationsunterschieds des zu regelnden Stoffes hinterlegt wird. Mittels eines Modells zur Abbildung der Stoffübertrittseigenschaften über den semipermeablen Filter wird die Konzentrationsänderung des zu regelnden Stoffes im vaskulären Blutkreislauf des Patienten bestimmt. Das Verfahren umfasst weiterhin die Möglichkeit, die Konzentration des zu regelnden Stoffes im Dialysatkreislauf stromaufwärts des Filters mit geeigneten Mitteln zu ändern und dahingehend anzupassen, dass die Konzentrationsänderung dieses Stoffes im vaskulären Blutkreislauf des Patienten zuvor eingegebenen Werten entspricht, bevorzugt einem Nullwert.

Es kann vorgesehen sein, dass die Konzentration des gelösten Stoffes im zweiten Teilkreislauf indirekt durch Bestimmung der Leitfähigkeit im ersten Teilkreislauf bestimmt wird.

Außerdem betrifft die Erfindung eine Blutbehandlungsvorrichtung mit den Merkmalen des Anspruchs 13. Danach ist vorgesehen, dass eine Blutbehandlungsvorrichtung aus einem extrakorporalen Blutkreislauf und einem Dialysatkreislauf besteht, wobei der Blutkreislauf und der Dialysatkreislauf durch wenigstens einen Filter semipermeabel voneinander getrennt sind, und einer Vorrichtung nach einem der Ansprüche 1 bis 12.

Weitere Einzelheiten und Vorteile sollen nun anhand eines in der Zeichnung näher dargestellten Ausführungsbeispiels erläutert werden.

Es zeigen:

Fig. 1:       eine schematische Darstellung einer Vorrichtung zur Natrium-Bilanzierung und Regelung,

Fig. 2a:     ein Diagramm mit Darstellung des Natriumkonzentrationsverlaufs eines simulierten Patienten mit erfindungsgemäßer Regelung,

Fig. 2b:     ein Diagramm mit Darstellung des Kaliumkonzentrationsverlaufs eines simulierten Patienten mit erfindungsgemäßer Regelung,

Fig. 3:       ein Diagramm mit Darstellung der Messkurven zur Natrium-Null-Bilanzregelung und

Fig. 4:       ein Diagramm mit Darstellung der Messkurven zum kontrollierten Natrium-Entzug.

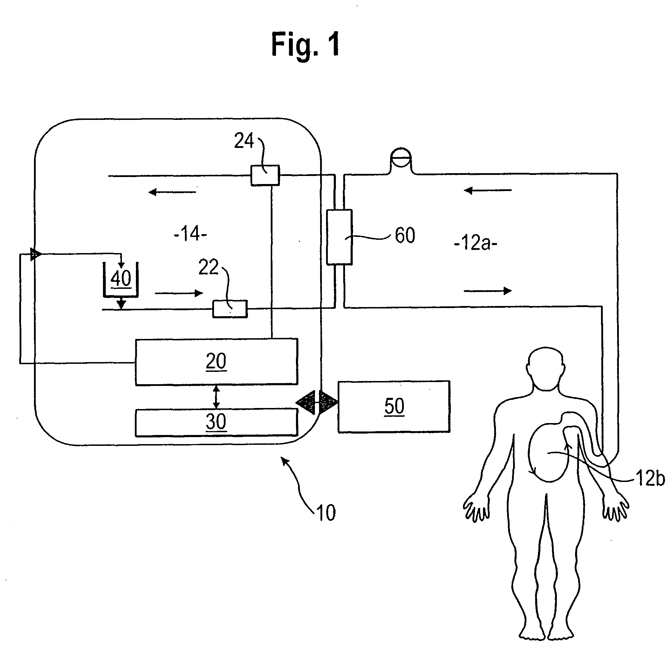

Fig. 1 zeigt eine Vorrichtung 10 zur Ermittlung und Regelung der Konzentration wenigstens eines gelösten Stoffes in einem Fluidkreislauf, im Einzelnen zur Natrium-regelung, -bilanzierung und -anzeige, die neben den für einen Dialysatkreislauf 14, der einem ersten Teilkreislauf 14 entspricht, nötigen Komponenten eine Detektionseinheit 20 mit als Messvorrichtungen 22, 24 zur Bestimmung der temperaturkompensierten Leitfähigkeit stromauf und stromab des Dialysators 60 ausgebildeten ersten und zweiten Mitteln 22, 24 sowie eine nicht näher dargestellte Vorrichtung zur genauen Bestimmung des Dialysatflusses (Bilanzkammer, Flussmesser usw.) umfasst. Ferner umfasst die Detektionseinheit 20 eine Regelungseinheit und nicht näher dargestellte Regelungs- und Speichermittel der Regelungseinheit, die eine Ermittlungseinheit zur dynamischen Ermittlung von Natrium im zweiten Teilkreislauf, bestehend aus dem extrakorporalen Blutkreislauf 12a und dem vaskulären Blutkreislauf 12b, ermöglichen. Grundsätzlich kann auch vorgesehen sein, dass die Detektionseinheit 20 und die Regelungseinheit baulich getrennt ausgeführt sind.

Die Daten der Leitfähigkeitssensoren 22, 24 werden von der Detektionseinheit 20 registriert und verarbeitet und die Werte für die Plasma-Natrium-Konzentration sowie die Natrium-Bilanz errechnet. Die Eingabe von Benutzervorgaben und Parametern der Regelung sowie die Ausgabe der errechneten Daten erfolgt über das Benutzerinterface 30. Die Detektionseinheit 20 enthält ferner weitere Regelungsmittel, die auf Basis der Messdaten und der Benutzervorgaben kontinuierlich Sollwerte an die Dosiereinheit 40 für das Dialysekonzentrat übermitteln.

Ferner ist eine Speichervorrichtung 50 zur Einspeicherung eines initialen Sollwertes für das Plasma-Natrium vorgesehen, wobei in die Speichervorrichtung 50 eine Chipkarte eingesteckt werden kann. Diese Chipkarte kann eine Patientenkarte sein und z. B. die Normwerte des Patienten eingespeichert vorhalten.

Die Ermittlungseinheit der Detektionseinheit 20 weist ein Regelungsmodell auf, das das Verhalten der gelösten Stoffe im zweiten Teilkreislauf 12a und 12b, das Übertrittsverhalten gelöster Stoffe über die semipermeable Membran des Dialysators 60 sowie deren zeitliche Änderung und/oder initiale Startwerte für die iterative Sollwertermittlung umfasst. Dabei ist vorgesehen, dass sich die Ermittlungseinheit dynamisch anpassen kann und z. B. Veränderungen in den Randbedingungen wie Konzentrationsverschiebungen aufnimmt.

Grundsätzlich gilt für das Regelungsmodell, dass die folgenden Gesetzmäßigkeiten hinterlegt sind bzw. das Regelungsmodell auf den folgenden Gesetzmäßigkeiten basiert und mit entsprechenden Berechnungsmitteln ausgestattet ist:

Die Massenbilanz bezogen auf den Strom eines Elektrolyten (=Massenfluss pro Zeiteinheit) $J_b$ aus dem Patienten lautet bzw. kann für den patientenseitigen Elektrolytstrom geschrieben werden:

$$\Delta J_b = \left(Q_b - Q_f\right)c_{bo} - Q_b c_{bi} = Q_b\left(c_{bo} - c_{bi}\right) - Q_f c_{bo}$$

(Gleichung 1)

Der dialysatseitige Elektrolytstrom $J_d$ ist:

$$\Delta J_d = \left(Q_d + Q_f\right)c_{do} - Q_d c_{di} = -Q_d\left(c_{di} - c_{do}\right) + Q_f c_{do}$$

Dabei sind $c_{bi}$ und $c_{bo}$ die blutseitigen Konzentrationen eines Elektrolyten vor bzw. nach dem Dialysator 60, $c_{di}$ und $c_{do}$ die entsprechenden dialysatseitigen Konzentrationen, $Q_b$ und $Q_f$ Blutfluss bzw. UF-Rate und $Q_d$ der Dialysatfluss inklusive Substituatfluss.

Blut und Dialysat stehen über die Dialysatormembranen in Verbindung, so dass wegen der Massenerhaltung im stationären Zustand gelten muss:

$$\Delta J_b + \Delta J_d = 0 \, .$$

Somit ergibt sich für die patientenseitige Massenbilanz bzw. instante Elektrolytbilanz bei der Dialyse, ausgedrückt durch dialysatseitige Größen:

$$\Delta J_b = Q_d c_{di} - \left(Q_d + Q_f\right)c_{do} = Q_d\left(c_{di} - c_{do}\right) - Q_f c_{do}$$

(Gleichung 3)

$\Delta J > 0$ bedeutet, dass ein Elektrolyt in den Patienten transferiert wird, $\Delta J < 0$, dass ihm dieser entzogen wird.

Für die Abspaltung des diffusiven Anteils wird vereinfachend angenommen, dass der konvektive Transport durch die Ultrafiltration die Ausgangskonzentration am Dialysator 60 nicht beeinflusst. Diese Annahme ist für kleine Moleküle und Ionen im Allgemeinen sehr gut erfüllt.

Dann gilt für die instante diffusive Elektrolytbilanz:

$$\Delta J_{diff} = Q_d\left(c_{di} - c_{do}\right)$$

(Gleichung 4)

Bei der Berechnung der Elektrolytbilanz muss berücksichtigt werden, dass zwischen Eintritt von frischem Dialysat mit der Konzentration $C_{do}$ eine Verzögerungszeit $t_f$ vergeht. Diese hängt von Maschinenhydraulik, Dialysatorvolumen und Dialysatfluss ab.

Die zwischen Dialysat und Patient transferierte Elektrolytmenge erhält man unter Berücksichtigung der Verzögerungszeit durch Integration (kumulative Bilanz unter Berücksichtigung der Verzögerungszeit):

$$M_{diff}(t) = \int_{\tau=0}^{t} \Delta J_{diff}(\tau)d\tau = \int_{\tau=0}^{t} c_{di}(\tau - t_f)Q_d(\tau - t_f)d\tau - \int_{\tau=0}^{t} c_{do}(\tau)Q_d(\tau)d\tau$$

$$M_{ges}(t) = M_{diff}(t) - \int_{\tau=0}^{t} c_{do}(\tau)Q_f(\tau)d\tau$$

(Gleichung 5)

Ist das Verteilungsvolumen $V$ des Patienten bekannt, so kann im 1-Pool-Modell jederzeit abgeschätzt werden, um welchen Betrag $dc_{Pat}$ sich die mittlere Elektrolytkonzentration im Patienten geändert hat. Diese Änderung wäre dann nach einer hinreichenden Äquilibrierungszeit auch im Patientenplasma zu beobachten (Konzentrationsänderung im Patienten):

$$dc_{Pat}(t) = \frac{M_{Diff}(t)}{V}$$

(Gleichung 6)

Der diffusive Strom $\Delta J_{j,diff}$ einer Ionenart $j$ zwischen Blut und Dialysat wird bestimmt durch die um den stoffspezifischen Donnanfaktor $\alpha_j$ korrigierten Konzentrationsdifferenz zwischen Blut und Dialysat und der stoffspezifischen Clearance $D_j$ (Diffusionsstrom zwischen Blut und Dialysat):

$$\Delta J_{j,diff}(t) = Q_d\left(c_{j,di}(t) - c_{j,do}(t)\right) = D_j\left(\frac{c_{j,di}(t)}{\alpha_j} - c_{j,bi}(t)\right)$$

(Gleichung 7)

Der Diffusionsstrom führt zu einer Konzentrationsänderung im Patienten, die zudem von der stoffspezifischen Anzahl und Größe der Kompartimente abhängt, in denen sich der Stoff verteilt.

Da im folgenden der Konzentrationsverlauf im Patienten nur zu einer Leitfähigkeits-Korrektur auf der Dialysatseite verwendet wird, ist im Rahmen der benötigten Genauigkeit eine gute Näherung im 1-Pool-Modell möglich.

In diesem Modell führt $\Delta J_{j,diff}$ im Patienten mit Verteilungsvolumen $V$ zu einer differenziellen Konzentrationsänderung $dc_{j,bi}$ :

$$dc_{j,bi} = \frac{\Delta J_{j,diff}}{V} dt .$$

Somit kann der zeitliche Verlauf der Konzentration im Patienten $c_{j,bi}$ bei bekannter Ausgangskonzentration $c_{j,o}$ fortlaufend berechnet werden (Berechnung des Verlaufs der Ionenkonzentration im Patienten):

$$c_{j,bi}\left(t_0\right) = c_{j,0}$$

$$c_{j,bi}\left(t_n\right) = c_{j,bi}\left(t_{n-1}\right) + \frac{\Delta J_{j,diff}(t)}{V}\left(t_n - t_{n-1}\right).$$

(Gleichung 8)

Die Plasma-Natrium-Konzentration kann auf der Grundlage einer Messung der Leitfähigkeit des Dialysats stromauf- und stromabwärts des Dialysators 60, der Dialysance $D$ sowie der Dialysat-, Substituat- und Ultrafiltrationsflüsse berechnet werden. Im Einzelnen gilt für die Plasmaleitfähigkeit:

$$\sigma_{bi} = \sigma_{di} + \frac{Q_d + Q_f + Q_s}{D}\left(\sigma_{do} - \sigma_{di}\right)$$

(Gleichung 9)

Die Ergebnisse der Regelung sowie Benutzereinstellungen vor Beginn oder während der Regelung können intern oder extern (Patientenkarte, Netzwerk mit Datenbanksystem) patientenindividuell gespeichert werden.

Im Allgemeinen ist die temperaturkompensierte Leitfähigkeit $\sigma$ einer Lösung eine Funktion der Konzentration aller ihrer Bestandteile. Im Bereich der in der Praxis im Dialysat vorkommenden Konzentration kann sie als Linearkombination der Konzentrationen ihrer wesentlichen Bestandteile genähert werden (allgemeine Darstellung der Leitfähigkeit als Funktion der Stoffkonzentrationen):

$$\sigma = f\left(c_{Na^+}, c_{K^+}, \ldots\right) = \sum_j c_j \gamma_j + \sigma_{Ofs}$$

(Gleichung 10)

Hierin sind $c_j$ die Ionenkonzentrationen und $\gamma_j$ deren bekannte molare Leitfähigkeiten bei typischen Dialysatzusammensetzungen und $\sigma_{Ofs}$ das Offset aus der Linearisierung. Da die Ladungsneutralität $\sum_j c_j z_j = 0$ ($z_j$ Wertigkeit des Ions $j$) gewährleistet sein muss, lässt sich die Konzentration der Chloridionen mit $z_{Cl^-} = -1$ eliminieren:

$$c_{Cl^-} = \sum_{j \neq Cl^-} c_j \gamma_j \Rightarrow \sigma = \sum_{j \neq Cl^-} c_j \left(\gamma_j + z_j \gamma_{Cl^-}\right) + \sigma_{Ofs}$$

Bei einer typischen Dialysatzusammensetzung gilt also für die Leitfähigkeit (Linearisierte Umrechnung zwischen Stoffkonzentration und Leitfähigkeit) mit $\tilde{\gamma}_j = \gamma_j + z_j \gamma_{Cl^-}$ :

$$\sigma = c_{Na^+}\tilde{\gamma}_{Na^+} + c_{K^+}\tilde{\gamma}_{K^+} + c_{Ca^{++}}\tilde{\gamma}_{Ca^{++}} + c_{Mg^{++}}\tilde{\gamma}_{Mg^{++}} + c_{Bic^-}\tilde{\gamma}_{Bic^-} + c_{Ac^-}\tilde{\gamma}_{Ac^-} + c_{Glu}\tilde{\gamma}_{Glu} + \sigma_{Ofs}$$

(Gleichung 11)

Die Koeffizienten $\tilde{\gamma}_j$ können empirisch aus Messdaten bestimmt werden. Dabei sind auch Koeffizienten $\gamma_j < 0$ etwa für Glucose und Harnstoff denkbar, was darauf zurückzuführen ist, dass nicht ionogene Stoffe die Ionenbewegung stören und dadurch die Leitfähigkeit verringern.

Mittels der in der Dialysemaschine verwendeten Leitfähigkeits-Sensoren 22, 24 kann sehr genau eine Leitfähigkeit-Bilanz bestimmt werden.

Aus dieser kann dann eine Natrium-Bilanz abgeschätzt werden, wenn aus der Leitfähigkeit-Änderung zwischen Dialysatoreingang und -ausgang die Änderung der Natrium-Konzentration berechnet werden kann. Hierzu muss der Beitrag der Konzentrationsänderung der übrigen Dialysatbestandteile zur Leitfähigkeits-Änderung abgeschätzt werden, wozu als Näherung folgendes Modell verwendet wird:

1. Neben Na⁺-Ionen sowie den implizit berücksichtigten Cl⁻ -Ionen tragen im wesentlichen Konzentrationsänderungen der K⁺- und Bic⁻-Ionen zur Leitfähigkeit-Änderung bei, da sich im allgemeinen die Konzentration dieser Ionen im Verlauf einer Dialyse am stärksten im Patienten ändert.

2. Die Änderung der Konzentration der übrigen Dialysatbestandteile hat keinen nennenswerten Einfluss auf die Leitfähigkeit-Änderung, sei es, weil die Konzentrationsänderungen zu gering sind oder sich die durch die Konzentrationsänderungen der einzelnen Bestandteile bewirkten Leitfähigkeits-Änderungen gegenseitig kompensieren. Ihre Konzentration kann daher als konstant angenommen werden (s. u.).

Damit kann basierend auf Gleichung 11 die Leitfähigkeit $\sigma_{di}$ am Dialysatoreingang angegeben werden:

$$\sigma_{di} = \tilde{\sigma}_{ofs}^{(di)} + \tilde{\gamma}_{Na^+}\, c_{Na^+}^{(di)}$$

mit

$$\tilde{\sigma}_{ofs}^{(di)} = c_{K^+}^{(di)}\, \tilde{\gamma}_{K^+} + c_{Ca^{++}}^{(di)}\, \tilde{\gamma}_{Ca^{++}} + c_{Mg^{++}}^{(di)}\, \tilde{\gamma}_{Mg^{++}} + c_{Bic^-}^{(di)}\, \tilde{\gamma}_{Bic^-} + c_{Ac^-}^{(di)}\, \tilde{\gamma}_{Ac^-} + c_{Glu}^{(di)}\, \tilde{\gamma}_{Glu} + \sigma_{Ofs}$$

Die Konstante $\tilde{\sigma}_{Ofs}$ enthält den Beitrag aller Bestandteile des frischen Dialysats außer Na⁺. Analog gilt für die Leitfähigkeit $\sigma_{do}$ am Dialysatorausgang:

$$\sigma_{do} = \tilde{\sigma}_{ofs}^{(do)} + \tilde{\gamma}_{Na^+}\, c_{Na^+}^{(do)}$$

Gemäß der obigen Annahmen lässt sich $\tilde{\sigma}_{ofs}^{(do)}$ schreiben als

$$\tilde{\sigma}_{ofs}^{(do)} = \tilde{\sigma}_{ofs}^{(di)} + \Delta\sigma_{do}$$

Der Beitrag der Konzentrationsänderung von K⁺- und Bic⁻ ist dann in $\Delta\sigma_{do}$ enthalten (Leitfähigkeits-Kompensation am Dialysatorausgang):

$$\Delta\sigma_{do}(t) = -\frac{1}{Q_d}\left(\Delta J_{K,diff}(t)\,\tilde{\gamma}_K + \Delta J_{Bic^-,diff}(t)\,\tilde{\gamma}_{Bic^-}\right)$$

(Gleichung 12)

Hierbei können die $\Delta J_{j,diff}$ iterativ mittels Gleichung 8 und Gleichung 7 bestimmt werden, wobei j stellvertretend für K und Bic⁻ steht. Einsetzen der Natrium-Konzentrationen aus obigen Ausdrücken für $\sigma_{di}$ und $\sigma_{do}$ in Gleichung 5 ergibt dann unter der Annahme, dass sich die Bic- und K- Konzentration in der Zeit $t_f$ nicht ändert, für die diffusive Natrium-Bilanz (Leitfähigkeitsbasierte diffusive Natrium-Bilanz):

$$M_{diff}^{(Na)}(t) = \frac{1}{\tilde{\gamma}_{Na^+}}\left[\int_{\tau=0}^{t}\sigma_{dr}(\tau - t_f)Q_d(\tau - t_f)d\tau - \int_{\tau=0}^{t}\sigma_{do}(\tau) - \Delta\sigma_{do}(\tau))Q_d(\tau)d\tau\right] = M_{diff}^{(LF)}(t) + M_{diff}^{(IC)}(t)$$

$$M_{diff}^{(IC)}(t) = \frac{1}{\tilde{\gamma}_{Na^+}}\left[\int_{\tau=0}^{t}\Delta\sigma_{do}(\tau)Q_d(\tau)d\tau\right]$$

$$M_{diff}^{(LF)}(t) = \frac{1}{\tilde{\gamma}_{Na^+}}\left[\int_{\tau=0}^{t}\sigma_{di}(\tau - t_f)Q_d(\tau - t_f)d\tau - \int_{\tau=0}^{t}\sigma_{do}(\tau)Q_d(\tau)d\tau\right]$$

(Gleichung 13)

Damit lässt sich die diffusive Natrium-Bilanz zerlegen in eine Leitfähigkeits-Bilanz $M_{diff}^{(LF)}\tilde{\gamma}_{Na^+}$, die direkt aus der Leitfähigkeits-Messung vor und hinter dem Dialysator 60 bestimmt wird, und einen Korrekturterm für den Ionenaustausch über die Dialysatormembran $M_{diff}^{(IC)}\tilde{\gamma}_{Na^+}$, der mittels eines Patienten- und Dialysatormoduls berechnet wird.

Aufgabe der Regelung ist es, bis zum Ende der Dialyse die vom Benutzer vorgegebene diffusive Natrium-Bilanz im Patienten zu erreichen. Bei Anwendung der Setpoint-Hypothese ist das Ziel eine isonaträmische Dialyse, also eine Natrium-Nullbilanz. Soll von der Nullbilanz abgewichen werden, so kann der Benutzer initial eine zusätzliche Natrium-Menge $M_{diff}^{(User)}(t_{End})$ vorgeben, die diffusiv bis zum En-de der Behandlung transferiert werden soll. $M_{diff}^{(User)}$ ist >0, wenn dem Patienten NaCl zugeführt werden soll, <0, wenn ihm NaCl entzogen werden soll. Die Natrium-Regelung verteilt diese Menge linear auf den Zeitraum bis zu 30 min vor Ende der UF-Zeit ($t_{End} = t_{UF}$ - 30 min):

$$M_{diff}^{(User)}(t) = \begin{cases} M_{diff}^{(User)}(t_{End})\frac{t}{t_{End}} & t \leq t_{End} \\ M_{diff}^{(User)}(t_{End})t & t > t_{End} \end{cases}$$

(Gleichung 14)

Dabei wird davon ausgegangen, dass dieser zusätzliche Natrium-Transfer den Austausch der übrigen Ionen nicht beeinflusst, auch wenn die Regelung zur Realisierung von denjenigen Konzentrateinstellungen abweicht, die zum Erreichen der Natrium-Nullbilanz vorgenommen würden.

Zum Erreichen des Bilanzierungsziels muss die Regelung zwei Bedingungen erfüllen:

1. Der diffusive Natrium-Fluss zwischen Patient und Dialysat (s. Gleichung 4) muss verschwinden. Unter Berücksichtigung einer Dialysatlaufzeit $t_f$ zwischen den beiden Leitfähigkeits-Sensoren und der Leitfähigkeit-Kompensation für die Ionenverschiebung ist dieses äquivalent zur Forderung, dass gilt:

$$\sigma_{do}(t) - \Delta\sigma_{do}(t) = \sigma_{di}(t - t_f)$$

2. Zugleich soll die Benutzervorgabe für die diffusive Bilanz zu jedem Zeitpunkt erfüllt sein, wobei auch eine akkumulierte Fehlbilanz ausgeglichen werden muss. Daher muss zusätzlich gelten (vgl. Gleichung 13):

$$M_{diff}(t) = M_{diff}^{(User)}(t).$$

Diese Forderungen werden in der Ermittlungseinheit bzw. im Regelungsmodell durch einen PI-Regler mit Totzeit realisiert. Dieser berechnet zunächst aus den gemessenen Leitfähigkeit die aktuell einzustellende Soll-Leitfähigkeit:

$$\sigma_{Soll}(t) = \sigma_{di}(t - t_f) + F_2\, \tilde{\gamma}_{Na^+}\left((\sigma_{do}(t) - \Delta\sigma_{do}(t)) - \sigma_{di}(t - t_f)\right) - F_1\left(M_{diff}(t) - M_{diff}^{(User)}(t)\right)$$

(Gleichung 15)

Die Verzögerungszeit $t_f$ wird so bestimmt, dass sich das aktuell in der stromabwärts gelegenen Leitfähigkeit-Zelle 24 befindliche Volumenelement vor der Zeit $t_f$ in der stromaufwärts gelegenen Leitfähigkeit-Zelle 22 befunden hat. Die Stellgröße des Reglers in Gleichung 15 hängt also von den gemessenen Leitfähigkeit an Dialysatoreingang und -ausgang sowie von Korrekturtermen eines Modells ab, dessen Parameter initial festgelegt werden. F1 und F2 sind Reglerkonstanten, die empirisch oder analytisch mit dem Fachmann bekannten Methoden der Systemtheorie derart bestimmbar sind, dass sich ein möglichst gutes Regelverhalten, insbesondere im Hinblick auf die Sprungantwort, ergibt.

Zunächst erfolgt die Berechnung einer Plasma-Äquivalent-Leitfähigkeit $\sigma_{bi}$ aus den gemessenen Leitfähigkeit vor und hinter dem Dialysator 60 nach Gleichung 9.

Hieraus wird dann als Umkehrung von Gleichung 11 die Natrium-Konzentration berechnet (Berechung des Plasma-Natrium):

$$c_{Na^+} = \frac{1}{\tilde{\gamma}_{Na^+}}\left(\sigma_{bi} - c_{K^+}\tilde{\gamma}_{K^+} - c_{Ca^{++}}\tilde{\gamma}_{Ca^{++}} - c_{Mg^{++}}\tilde{\gamma}_{Mg^{++}} - c_{Bic^-}\tilde{\gamma}_{Bic^-} - c_{Ac^-}\tilde{\gamma}_{Ac^-} - c_{Glu}\tilde{\gamma}_{Glu} - \sigma_{Ofs}\right)$$

(Gleichung 16)

Dabei werden die Konzentrationen von Kalium und Bicarbonat wie in Gleichung 8 beschrieben mittels eines 1-Pool-Patientenmodells abgeschätzt. Für die übrigen Dialysatbestandteile werden folgende Konzentrationen als konstant angenommen:

$$c_{Ca^{++}} = 1{,}2\,mmol/l,\, c_{Mg^{++}} = 0{,}5\,mmol/l,\, c_{Ac^-} - 2{,}0\,mmol/l,\, c_{Glu} = 1{,}2\,g/l$$

Um die nächste Dialyse bereits von Beginn an mit demjenigen Dialysat-Natrium $c_{di}^{(ZBal)}$ starten zu können, das für diesen Patienten zum Erreichen der Natrium-Nullbilanz nötig ist, kann dieser Wert von der Regelung ermittelt und am Ende der Dialyse auf der Patientenkarte gespeichert werden.

Wurde die diffusive Natrium-Nullbilanz erreicht, so ist $c_{di}^{(ZBal)}$ meist gleich dem am Ende von der Regelung eingestellten Natrium-Sollwert. Jedoch kann es sein, dass keine Nullbilanz erreicht wurde, z. B. weil vom Benutzer eine Natrium-Verschiebung gewählt wurde oder der zum Erreichen der Nullbilanz nötige Wert innerhalb der Fenstergrenzen nicht eingestellt werden konnte. Ebenso kann es sein, dass zwar am Ende der Dialysebehandlung die Nullbilanz erreicht wurde, zum Ausgleich einer Fehlbilanz aber der letzte Natrium-Sollwert von demjenigen abweicht, der einer Natrium-Nullbilanz entsprechen würde.

Daher wird $c_{di}^{(ZBal)}$ über ein Modell genähert: Hierbei wird vereinfachend angenommen, dass sich der Patient durch ein Verteilungsvolumen $V$ beschreiben lässt, das gegen eine mittlere Dialysatkonzentration $\langle c_{di} \rangle$ dialysiert wird mit

$$\langle c_{di} \rangle = \int_0^T Q_d(t) c_{di}(t) dt \Big/ \int_0^T Q_d(t) dt \; .$$

(Gleichung 17)

Aus Gleichung 6 und Gleichung 7 folgt unter Vernachlässigung des Donnan-Effekts:

$$c_{bi}(t) = \langle c_{di} \rangle + (c_{bi}(0) - \langle c_{di} \rangle) \exp(-\frac{D}{V} t)$$

$$M_{Diff}(t) = V * (c_{bi}(0) - c_{bi}(t)) - V \left( 1 - \exp(-\frac{D}{V} t) \right)(c_{bi}(0) - \langle c_{di} \rangle)$$

Eine Natrium-Nullbilanz ($M_{Diff}(t) = 0$) wird genau dann erreicht, wenn $c_{di}^{(ZBal)} = c_{bi}(0)$ ist. Daraus folgt für $c_{di}^{(ZBal)}$

$$c_{di}^{(ZBal)} = \langle c_{di} \rangle - \frac{M_{Diff}(t)}{V \left( 1 - \exp(-\frac{D}{V} t) \right)}$$

(Gleichung 18)

Aus Gleichung 18 kann somit der Sollwert des Natriumgehalts des Dialysats für isonaträmische Dialyse ermittelt und auf die Patientenkarte eingespeichert werden. Die Detektionseinheit 20 und die Speichervorrichtung 50, in die die Patientenkarte(n) einsteckbar ist, sind hierzu mit Datenaustauschmitteln verbunden.

Der Anwender gibt vor Beginn der Dialyse den Regelbereich vor. Dieses kann ein im Zentrum üblicher Wert für das Dialysat-Natrium, ein Schätzwert für das zum Erreichen der Isonaträmie benötigte Dialysat-Natrium, eine Labormessung des Patientennatriums oder ein aus früheren Behandlungen durch die Regelung ermittelter Wert sein. Aus Sicherheitsgründen ist es der Regelung während der Behandlung nur erlaubt, von diesem Wert nur innerhalb eines festen Toleranzbereichs, z. B. +/-5 % ohne Nachfrage beim Benutzer abzuweichen. Sind zum Erreichen der Isonaträmie größere Abweichungen vom Vorgabewert nötig, so wird der Benutzer zu Änderungen des Vorgabewerts aufgefordert. Zur Anwendung des Modells zur Korrektur der diffusiven Bilanz ist zudem das Verteilungsvolumen des Patienten einzugeben. Dieses kann mittels einer anthropometrischen Formel berechnet werden oder mittels Bioimpedanz oder "kinetic modelling" bestimmt werden. Weiterhin werden die initialen Konzentrationen von Bicarbonat und Kalium im Patienten benötigt. Diese können aus einer Analyse mittels Blutgasanalysator vor Beginn der Dialysebehandlung stammen.

Alle benötigten Parameter (Verteilungsvolumen, initiales Dialysat-Natrium, Initialwerte für Bicarbonat und Kalium) sind häufig für einen Patienten zu Beginn der Dialysebehandlung weitgehend gleich und können daher auf einer Patientenkarte gespeichert und automatisiert zu Beginn der Behandlung eingelesen werden.

Nach Start der Behandlung werden kontinuierlich der Dialysatfluss sowie die Leitfähigkeit stromauf und stromab des Dialysators 60 bestimmt. Des Weiteren erfolgt die Berechnung der aktuellen Bicarbonat- und Kalium-Konzentration nach Gleichung 7 und Gleichung 8, wobei angenommen wird, dass die Kalium-Clearance der Natrium-Clearance entspricht, und dass die Bicarbonat-Clearance 70 % der Natrium-Clearance entspricht. Bis zum Vorliegen der ersten Clearancemessung (nach ca. 20 min) wird die Natrium-Clearance aus dem Blutfluss abgeschätzt.

Aus diesen Daten erfolgt dann gemäß Gleichung 12 und Gleichung 13 die Berechnung der Leitfähigkeits-Bilanz und des Korrekturterm für den Ionenaustausch und somit für die Natrium-Bilanz.

Die stromauf und stromab gemessenen Dialysat-Leitfähigkeit, die Natrium-Bilanz sowie der Korrekturterm für die Dialysat-Leitfähigkeit stromab des Dialysators 60 (s. Gleichung 12) sind dann die Eingangsgrößen für die Natrium-Regelung gemäß Gleichung 15. Die so bestimmte Soll-Leitfähigkeit wird schließlich unter Berücksichtigung der Zusammensetzung des Dialysekonzentrats in einen Sollwert für das Dialysat-Natrium umgerechnet und dieser Vorgabewert an die Dosiereinheit 40 übermittelt.

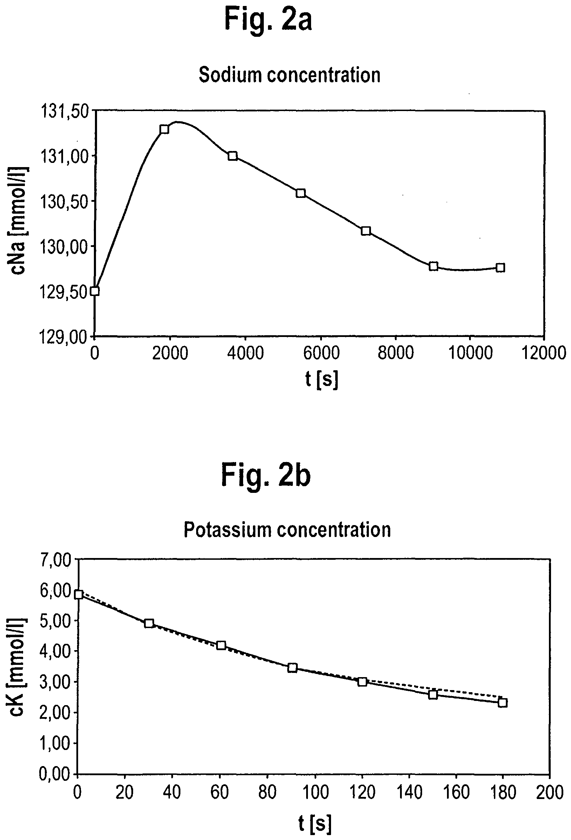

Fig. 2a und 2b zeigen die Verläufe der Natrium- und Kaliumkonzentration während einer Dialysebehandlung für einen künstlichen Patienten, der durch einen Behälter mit 20l Salzlösung simuliert wurde, unter Verwendung der erfindungsgemäßen Regelung für die Natriumkonzentration. Hierbei sind die Startkonzentrationen im Patienten 6 mmol/l Kalium und 130 mmol/l Natrium, für das Dialysat 2 mmol/l Kalium und 145 mmol/l Natrium. Die erfindungsgemäße Regelung passt die Dialysatzusammensetzung so an, dass sich die Natriumkonzentration über die Dialysedauer möglichst wenig ändert, wie in Figur 2a gezeigt. Figur 2b zeigt den Verlauf der Kaliumkonzentration im Patienten, wobei die durchgezogene Linie Messwerte und die gestrichelte Linie die vom Modell berechneten Werte darstellen. Eine Regelung, die nur die Leitfähigkeit des Dialysats konstant hält, würde bei dem vorliegenden Beispiel den Verlust von ca. 3,5 mmol/l Kalium durch die Zufuhr von ca. 4,5 mmol/l Natrium kompensieren, was zu einem unerwünschten Anstieg der Natriumkonzentration im Patienten führen würde. Die erfindungsgemäße Regelung hält die Natriumkonzentration im Patienten annähernd konstant, obwohl die Konzentrationen von Natrium und Kalium im Dialysat zu Beginn der Dialyse stark von der im Blut des Patienten abweichen.

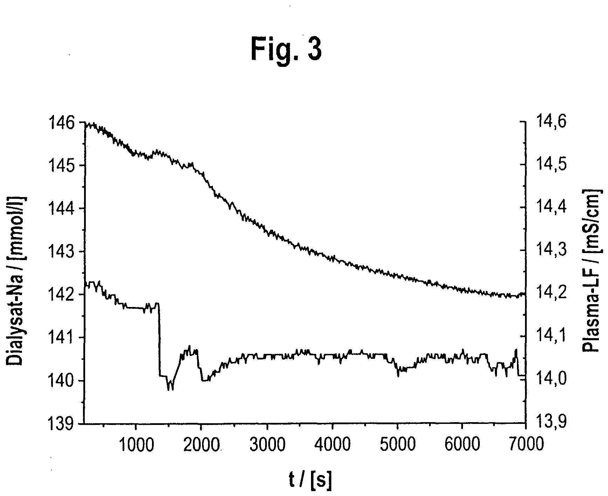

Fig. 3 zeigt einen in-vitro Laborversuch zu Natrium-Nullbilanzregelung unter Verwendung der Kompensation für den Austausch von Kalium und Bicarbonat. Dargestellt sind der Sollwert der Regelung für das Dialysat-Natrium (untere Kurve, linke Skala) sowie der gemessene Verlauf der Leitfähigkeit im Modellpatienten (obere Kurve, rechte Skala). Der Modellpatient (Verteilungsvolumen 10l) besaß zu Beginn der Leitfähigkeit von 14,6 mS/cm. Ohne Kompensation hätte daher die Regelung bei der vorliegenden Dialysatzusammensetzung ein Dialysat-Natrium von ca. 146 mmol/l eingestellt und die Leitfähigkeit konstant gehalten. Übertragen auf einen Patienten wäre dabei der Leitfähigkeitsabfall durch Kaliumverlust und Aufnahme von Bicarbonat durch eine Zufuhr von Natrium kompensiert worden, so dass das End-Natrium deutlich höher gelegen hätte als das Start-Natrium.

Durch Verwendung der Kompensation stellt die Regelung schon zu Beginn der Behandlung einen deutlich niedrigeren Natriumwert ein und hält diesen weitgehend konstant. Der Abfall der Leitfähigkeit des Patienten entspricht der Situation, dass zwar der Wert des Plasma-Natriums konstant bleibt, die Plasma-Leitfähigkeit aber durch Kaliumverlust und Aufnahme von Bicarbonat sinkt.

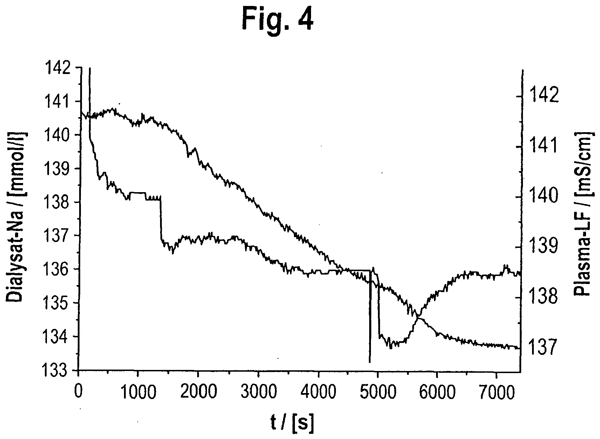

Fig. 4 zeigt einen in-vitro Laborversuch zum benutzerdefinierten Entzug von NaCl. Im Gegensatz zum Stand der Technik, nach dem lediglich die Regelung des Plasma-Natrium auf einen vorgegebenen Wert möglich ist, kann der Anwender relative Änderungen zum aktuellen Wert des Plasma-Natriums veranlassen, d.h. angeben, wieweit von der Isonatriämie abgewichen werden soll. Hierzu muss das Verteilungsvolumen des Patienten eingegeben bzw. von einem Datenträger gelesen werden. Zudem muss die Behandlungsdauer, typischerweise als Dauer der Ultrafiltration, angegeben werden. Der Benutzer kann nun einen Sollwert für die Natrium-Konzentrationsänderung in mmol/l angeben. Ebenso werden die Initialwerte von Kalium und Bicarbonat benötigt, damit die vorstehend beschriebene Korrektur der Bilanzierung durchgeführt werden kann.

Diese wird dann von der Regelung wie ebenfalls vorstehend beschrieben linear auf die geplante Behandlungsdauer verteilt. Hierbei werden im Behandlungsverlauf Änderungen der Clearance und durch den Ionenaustausch bedinge Änderungen der Leitfähigkeit automatisch berücksichtigt. Dieses führt zu einer Behandlung mit weitgehend konstantem Difffusionsgradienten zwischen Plasma und Dialysat. Alternativ kann der Benutzer auch eine Gesamt-Natrium-Menge vorgeben, die während der Dialyse durch Ultrafiltration und durch Diffusion dem Patienten entzogen bzw. zugeführt werden soll. Dieses ist für Anwendungen nützlich, in denen die Salzaufnahme zwischen den Dialysen durch die Diät genau bekannt ist.

Fig. 4 zeigt einen in-vitro Laborversuch zum benutzerdefinierten Entzug von NaCl (Benutzervorgabe 4,5 mmol/l) aus einem Modellpatienten (NaCl-Lösung, Verteilungsvolumen 10 l). Dabei illustriert die im linken Teil unterhalb verlaufende Kurve den Sollwert der Regelung für das Dialysat-Natrium und die oberhalb verlaufende Kurve den gemessene Verlauf der Leitfähigkeit im Modellpatienten. Im Versuch wurde keine Kompensation für den Ionenaustausch durchgeführt, so dass der durch die Regelung eingestellte Verlauf des Dialysat-Natriums allein durch den Algorithmus zur Erreichung der Konzentrationsverschiebung hervorgerufen wurde. Der konstante Wert des Dialysat-Natriums bei ca. 3500 s kommt dadurch zustande, dass zu diesem Zeitpunkt das untere Ende des Regelbereichs erreicht war. Erst bei 5000 s wurde dieser durch den Benutzer zu niedrigeren Natrium-Konzentrationen hin verschoben, weshalb in der Folge ein "Nachholen" des geplanten Salzentzugs erfolgte.

**Patentansprüche**

1. Vorrichtung (10) zur Ermittlung und Regelung der Konzentration wenigstens eines gelösten Stoffes in einem Fluidkreislauf, wobei der Fluidkreislauf aus einem extrakorporalen Blutkreislauf (12a) und einem Dialysatkreislauf (14) mit wenigstens einem Filter (60), der die Teilkreisläufe (12a, 14) semipermeabel trennt, besteht, wobei die Vorrichtung eine Detektionseinheit (20) mit wenigstens einem Mittel (24), mit dem der Konzentrationsunterschied wenigstens eines ersten gelösten Stoffes vor und nach dem Filter (60) in dem Dialysatkreislauf (14) mittelbar und/oder unmittelbar durch die Detektionseinheit (20) bestimmbar ist, aufweist, und wobei die Vorrichtung eine Regelungseinheit zur Regelung der Konzentration des wenigstens einen gelösten Stoffes in dem Fluidkreislauf aufweist,
**dadurch gekennzeichnet,**
**dass** die Vorrichtung ferner eine Ermittlungseinheit aufweist, die unter Vorhaltung eines Modells den Einfluss wenigstens eines zweiten gelösten Stoffes, der nicht identisch zum wenigstens einen ersten gelösten Stoff ist, auf die durch das Mittel (24) der Detektionseinheit (20) erfolgende Bestimmung des Konzentrationsunterschiedes bestimmt, und den Konzentrationsunterschied des wenigstens einen ersten gelösten Stoffes vor und nach dem Filter (60) im Dialysatkreislauf (14) berechnet.

2. Vorrichtung (10) nach Anspruch 1, **dadurch gekennzeichnet, dass** die Regelungseinheit Regelungsmittel und Speichermittel umfasst, wobei durch die Regelungsmittel der Istwert des ermittelten Konzentrationsunterschieds des wenigstens einen ersten gelösten Stoffes im Dialysatkreislauf (14) gegen einen Sollwert für die Konzentration oder für die Konzentrationsänderung dieses Stoffes im extrakorporalen Blutkreislauf (12a) oder im vaskulären Blutkreislauf (12b) abgleichbar ist und die Konzentration des wenigstens einen ersten gelösten Stoffes im Dialysatkreislauf (14) auf einen Sollwert mittels der Regelungsmittel einregelbar ist oder wobei durch die Regelungsmittel aus dem Istwert des ermittelten Konzentrationsunterschieds des wenigstens einen ersten gelösten Stoffes im Dialysatkreislauf (14) vor und nach dem Filter (60) ein Sollwert für die Konzentration dieses Stoffes im Dialysatkreislauf (14) stromaufwärts des Filters (60) ermittelbar ist und die Konzentration des wenigstens einen ersten gelösten Stoffes im Dialysatkreislauf (14) stromaufwärts des Filters (60) auf diesen Sollwert mittels der Regelungseinheit einregelbar ist.

3. Vorrichtung (10) nach Anspruch 1 oder 2, **dadurch gekennzeichnet, dass** die Detektionseinheit (20) zusätzlich zu dem ersten Mittel (24) wenigstens ein weiteres zweites Mittel (22) aufweist, mit dem der Konzentrationsunterschied des wenigstens einen ersten gelösten Stoffes vor und nach dem Filter (60) in dem Dialysatkreislauf (14) mittelbar und/oder unmittelbar durch die Detektionseinheit (20) bestimmbar ist, wobei ein Mittel (22) stromaufwärts und ein Mittel (24) stromabwärts des Filters (60) angeordnet ist.

4. Vorrichtung (10) nach einem der vorhergehenden Ansprüche, **dadurch gekennzeichnet, dass** die ersten und/oder zweiten Mittel (22, 24) Leitfähigkeitssensoren umfassen oder als solche ausgeführt sind.

5. Vorrichtung (10) nach Anspruch 4, **dadurch gekennzeichnet, dass** die Detektionseinheit (20) keine weiteren Mittel aufweist, mit denen im Blutkreislauf (12a) die Konzentration des wenigstens einen gelösten Stoffes bestimmbar ist oder der Sollwert für die Konzentration des wenigstens einen gelösten Stoffes im Dialysatkreislauf (14) stromaufwärts des Filters (60) bestimmbar ist.

6. Vorrichtung (10) nach Anspruch 4 oder 5, **dadurch gekennzeichnet, dass** durch die mittels der ersten und/oder zweiten Mittel (22, 24) ermittelte Leitfähigkeit mittelbar die Konzentration des wenigstens einen ersten gelösten Stoffes mittels der Regelungseinheit berechenbar ist.

7. Vorrichtung (10) nach einem der vorhergehenden Ansprüche, **dadurch gekennzeichnet, dass** die Konzentration des wenigstens einen ersten gelösten Stoffes im Dialysatkreislauf (14) stromaufwärts des Filters (60) mittels der Regelungsmittel auf den Sollwert einregelbar ist.

8. Vorrichtung (10) nach einem der Ansprüche 1 bis 6, **dadurch gekennzeichnet, dass** die Konzentration des wenigstens einen ersten gelösten Stoffes im Dialysatkreislauf (14) stromaufwärts des Filters (60) mittels der Regelungsmittel auf einen vom Sollwert abweichenden zweiten Sollwert einregelbar ist.

9. Vorrichtung (10) nach einem der vorhergehenden Ansprüche, **dadurch gekennzeichnet, dass** die Einregelung nur auf eine vordefinierte Schwankungsbreite im ersten oder zweiten Teilkreislauf (12a, 14) beschränkbar ist, vorzugsweise innerhalb von $\pm$ 5 % des Sollwertes.

EP 2 413 991 B1

**10.** Vorrichtung (10) nach einem der vorhergehenden Ansprüche, **dadurch gekennzeichnet, dass** eine Speichervorrichtung (50) zur Einspeicherung eines initialen Sollwertes für den gelösten Stoff vorgesehen ist, wobei die Speichervorrichtung (50) vorzugsweise ein entnehmbares Speichermedium umfasst.

**11.** Vorrichtung (10) nach Anspruch 10, **dadurch gekennzeichnet, dass** die Speichervorrichtung (50) mit der Regelungseinheit in Verbindung steht.

**12.** Vorrichtung (10) nach einem der vorhergehenden Ansprüche, **dadurch gekennzeichnet, dass** die Vorrichtung Bestandteil einer Dialysemaschine ist und/oder dass es sich bei dem gelösten Stoff um Natrium-Ionen handelt.

**13.** Blutbehandlungsvorrichtung bestehend aus einem extrakorporalen Blutkreislauf (12a) und einem Dialysatkreislauf (14), wobei der Blutkreislauf (12a) und der Dialysatkreislauf (14) durch wenigstens einen Filter (60) semipermeabel voneinander getrennt sind, und einer Vorrichtung nach einem der Ansprüche 1 bis 12.

**Claims**

**1.** An apparatus (10) for the determination and regulation of the concentration of at least one dissolved substance in a fluid circuit, wherein the fluid circuit comprises an extracorporeal blood circuit (12a) and a dialysate circuit (14) having at least one filter (60) which separates the partial circuits (12a, 14) in a semipermeable manner, wherein the apparatus has a detection unit (20) having at least one means (24) with which the concentration difference of at least one first dissolved substance can be determined indirectly and/or directly by the detection unit (20) before and after the filter (60) in the dialysate circuit (14); and wherein the apparatus has a regulation unit for regulating the concentration of the at least one dissolved substance in the fluid circuit,
**characterized in that**
the apparatus further has a determination unit which determines the influence of at least one second dissolved substance which is not identical to the at least one first dissolved substance on the determination of the concentration difference made by the means (24) of the detection unit (20) while using a model and calculates the concentration difference of the at least one first dissolved substance before and after the filter (60) in the dialysate circuit (14).

**2.** An apparatus (10) in accordance with claim 1, **characterized in that** the regulation unit comprises regulation means and storage means, wherein the actual value of the determined concentration difference of the at least one first dissolved substance in the dialysate circuit (14) can be calibrated by the regulation means with respect to a desired value for the concentration or for the concentration change of this substance in the extracorporeal blood circuit (12a) or in the vascular blood circuit (12b) and the concentration of the at least one first dissolved substance in the dialysate circuit (14) can be regulated to a desired value by means of the regulation means; or wherein a desired value for the concentration of this substance in the dialysate circuit (14) upstream of the filter (60) can be determined by the regulation means from the actual value of the determined concentration difference of the at least one first dissolved substance in the dialysate circuit (14) before and after the filter (60) and the concentration of the at least one first dissolved substance in the dialysate circuit (14) upstream of the filter (60) can be regulated to this desired value by means of the regulation unit.

**3.** A apparatus (10) in accordance with either of claims 1 or 2, **characterized in that** the detection unit (20) has, in addition to the first means (24), at least one further second means (22) with which the concentration difference of the at least one first dissolved substance can be determined indirectly and/or directly by the detection unit (20) before and after the filter (60) in the dialysate circuit (14), with one means (22) being arranged upstream and one means (24) being arranged downstream of the filter (60).

**4.** An apparatus (10) in accordance with one of the preceding claims, **characterized in that** the first and/or second means (22, 24) include conductivity sensors or are made as such.

**5.** An apparatus (10) in accordance with claim 4, **characterized in that** the detection unit (20) does not have any further means with which the concentration of the at least one dissolved substance can be determined in the blood circuit (12a); or **in that** the desired value for the concentration of the at least one dissolved substance can be determined in the dialysate circuit (14) upstream of the filter (60).

**6.** An apparatus (10) in accordance with either of claims 4 or 5, **characterized in that** the concentration of the at least one first dissolved substance can be calculated indirectly by the conductivity determined by means of the first and/or second means (22, 24) by means of the regulation unit.

7. An apparatus (10) in accordance with one of the preceding claims, **characterized in that** the concentration of the at least one first dissolved substance can be regulated to the desired value in the dialysate circuit (14) upstream of the filter (60) by means of the regulation means.

8. An apparatus (10) in accordance with one of the claims 1 to 6, **characterized in that** the concentration of the at least one first dissolved substance can be regulated to a second desired value differing from the desired value in the dialysate circuit (14) upstream of the filter (60) by means of the regulation means.

9. An apparatus (10) in accordance with one of the preceding claims, **characterized in that** the regulation can be limited only to a predefined fluctuation margin in the first or second partial circuits (12a, 14), preferably within $\pm 5\%$ of the desired value.

10. An apparatus (10) in accordance with one of the preceding claims, **characterized in that** a storage apparatus (50) is provided for the storing of an initial desired value for the dissolved substance, with the storage apparatus (50) preferably including a removable storage medium.

11. An apparatus (10) in accordance with claim 10, **characterized in that** the storage apparatus (50) is connected to the regulation unit.

12. An apparatus (10) in accordance with one of the preceding claims, **characterized in that** the apparatus is a component of a dialysis machine; and/or **in that** the dissolved substance represents sodium ions.

13. A blood treatment apparatus comprising an extracorporeal blood circuit (12a) and a dialysate circuit (14), wherein the blood circuit (12a) and the dialysate circuit (14) are separated from one another by at least one filter (60) in a semipermeable manner, and an apparatus in accordance with one of the claims 1 to 12.

**Revendications**

1. Dispositif (10) pour déterminer et réguler la concentration d'au moins une substance dissoute dans un circuit de fluide, le circuit de fluide étant constitué d'un circuit sanguin (12a) extracorporel et d'un circuit de dialysat (14), avec au moins un filtre (60), qui sépare les circuits partiels (12a, 14) de manière semi-perméable, le dispositif comportant une unité de détection (20) comprenant au moins un moyen (24), au moyen duquel la différence de concentration d'au moins une première substance dissoute avant et après le filtre (60) dans le circuit de dialysat (14) peut être déterminée indirectement et/ou directement par l'unité de détection (20), et le dispositif comportant une unité de régulation destinée à réguler la concentration de l'au moins une substance dissoute dans le circuit de fluide, **caractérisé en ce que** le dispositif comporte en outre une unité de détermination, qui détermine, en se référant à un modèle, l'influence d'au moins une seconde substance dissoute, qui n'est pas identique à l'au moins une première substance dissoute, sur la détermination de la différence de concentration effectuée par le moyen (24) de l'unité de détection (20), et calcule la différence de concentration de l'au moins une première substance dissoute avant et après le filtre (60) dans le circuit de dialysat (14).

2. Dispositif selon la revendication 1, **caractérisé en ce que** l'unité de régulation comprend des moyens de régulation et des moyens de stockage, les moyens de régulation permettant d'aligner la valeur réelle de la différence de concentration déterminée de l'au moins une première substance dissoute dans le circuit de dialysat (14) sur une valeur de consigne pour la concentration ou pour le changement de concentration de cette substance dans le circuit sanguin (12a) extracorporel ou dans le circuit sanguin (12b) vasculaire, et la concentration de l'au moins une première substance dissoute dans le circuit de dialysat (14) pouvant être réglée sur une valeur de consigne au moyen des moyens de régulation, ou les moyens de régulation permettant de déterminer, à partir de la valeur réelle de la différence de concentration déterminée de l'au moins une première substance dissoute dans le circuit de dialysat (14) avant et après le filtre (60), une valeur de consigne pour la concentration de cette substance dans le circuit de dialysat (14) en amont du filtre (60), et la concentration de l'au moins une première substance dissoute dans le circuit de dialysat (14) en amont du filtre (60) pouvant être réglée sur cette valeur de consigne au moyen de l'unité de régulation.

**3.** Dispositif (10) selon la revendication 1 ou 2, **caractérisé en ce que** l'unité de détection (20) comporte, en plus du premier moyen (24), au moins un autre, second moyen (22), au moyen duquel la différence de concentration de l'au moins une première substance dissoute avant et après le filtre (60) dans le circuit de dialysat (14) peut être déterminée indirectement et/ou directement par l'unité de détection (20), un moyen (22) étant disposé en amont et un moyen (24) en aval du filtre (60).

**4.** Dispositif (10) selon l'une des revendications précédentes, **caractérisé en ce que** le premier et/ou le second moyen (22, 24) comprend des capteurs de conductivité ou est exécuté en tant que tel.

**5.** Dispositif (10) selon la revendication 4, **caractérisé en ce que** l'unité de détection (20) ne comporte pas d'autres moyens, au moyen desquels la concentration de l'au moins une substance dissoute peut être déterminée dans le circuit sanguin (12a), ou la valeur de consigne pour la concentration de l'au moins une substance dissoute dans le circuit de dialysat (14) en amont du filtre (60) peut être déterminée.

**6.** Dispositif (10) selon la revendication 4 ou 5, **caractérisé en ce que** la concentration de l'au moins une première substance dissoute peut être calculée indirectement au moyen de l'unité de régulation grâce à la conductivité déterminée au moyen du premier et/ou du second moyen (22, 24).

**7.** Dispositif (10) selon l'une des revendications précédentes, **caractérisé en ce que** la concentration de l'au moins une première substance dissoute dans le circuit de dialysat (14) en amont du filtre (60) peut être réglée sur la valeur de consigne au moyen des moyens de régulation.

**8.** Dispositif (10) selon l'une des revendications 1 à 6, **caractérisé en ce que** la concentration de l'au moins une première substance dissoute dans le circuit de dialysat (14) en amont du filtre (60) peut être réglée sur une seconde valeur de consigne différente de la valeur de consigne au moyen des moyens de régulation.

**9.** Dispositif (10) selon l'une des revendications précédentes, **caractérisé en ce que** le réglage peut être limité uniquement à une marge de variation prédéfinie dans le premier ou le second circuit partiel (12a, 14), de préférence dans la plage de $\pm$ 5 % de la valeur de consigne.

**10.** Dispositif (10) selon l'une des revendications précédentes, **caractérisé en ce qu'**un dispositif de stockage (50) est prévu pour le stockage d'une valeur de consigne initiale pour la substance dissoute, le dispositif de stockage (50) comprenant de préférence un support de stockage amovible.

**11.** Dispositif (10) selon la revendication 10, **caractérisé en ce que** le dispositif de stockage (50) est en liaison avec l'unité de régulation.

**12.** Dispositif (10) selon l'une des revendications précédentes, **caractérisé en ce que** le dispositif fait partie d'un dialyseur et/ou **en ce que** la substance dissoute est des ions de sodium.

**13.** Dispositif de traitement du sang constitué d'un circuit sanguin (12a) extracorporel et d'un circuit de dialysat (14), le circuit sanguin (12a) et le circuit de dialysat (14) étant séparés l'un de l'autre de manière semi-perméable par au moins un filtre (60), et d'un dispositif selon l'une des revendications 1 à 12.

# Fig. 1

# Fig. 2a

## Sodium concentration

# Fig. 2b

## Potassium concentration

# Fig. 3

# Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4923613 A

- US 4508622 A

**In der Beschreibung aufgeführte Nicht-Patentliteratur**

- **PETITCLERC et al.** Sodium management in dialysis by conductivity. *Advances in renal replacement therapy,* Juli 1999, vol. 6 (3), 243-254